# EUROPEAN PATENT SPECIFICATION

(11) **EP 3 656 324 B1**
(45) Date of publication and mention of the grant of the patent: **21.01.2026**
(21) Application number: 18851209.9
(22) Date of filing: 19.02.2018
(51) Int. Cl.: A61B 18/12, A61B 18/14, A61B 18/00

(54) **ABLATION SYSTEM**
ABLATIONSSYSTEM
SYSTÈME D'ABLATION

(30) Priority: 29.08.2017 JP 2017164461
(43) Date of publication of application: 27.05.2020
(73) Proprietor: Japan Lifeline Co., Ltd., Tokyo 140-0002 (JP)
(72) Inventor: MIYAMOTO Hisao, Tokyo 140-0002 (JP)
(74) Representative: D Young & Co LLP
(86) International application number: PCT/JP2018/005775
(87) International publication number: WO 2019/043991

(56) References cited:
- EP-A2- 2 942 023
- JP-A- 2002 065 690
- JP-A- 2004 008 581
- JP-A- 2004 008 581
- JP-A- 2008 114 042
- JP-A- 2008 514 271
- CHENG YA-TING ET AL: "Percutaneous radiofrequency ablation for hepatocellular carcinoma: Early termination versus standard termination of ablation procedure", vol. 3, no. 4, 31 December 2015 (2015-12-31), pages 167 - 173, XP055780421, ISSN: 2351-9800, Retrieved from the Internet <URL:https://api.elsevier.com/content/article/PII:S2351979715001000?httpAccept=text/plain> [retrieved on 20210301], DOI: 10.1016/j.aidm.2015.07.001

## Description

### Technical Field

The invention relates to an ablation system that includes: an electrode needle to be percutaneously punctured into an affected site in a body; and a power supply device that supplies electric power directed to performing of an ablation (ablation).

### Background Art

As one of medical devices for treating an affected site (for example, an affected site having a tumor such as cancer) in a patient's body, an ablation system that performs an ablation on such an affected site has been proposed (for example, see Patent Literature 1). The ablation system includes: an electrode needle to be percutaneously punctured into the affected site in the body; and a power supply device that supplies electric power directed to performing of the ablation on the affected site.

### Citation list

### Patent Literature

Patent Literature 1: Japanese Patent No. 5907545
Other prior art is provided in EP2 942 023 A and Cheng Ya-Ting et al "Percutaneous Radio Frequency Ablation for Hepatocellular Carcinoma: Early Termination versus Standard Termination of Ablation Procedure". Advances in Digestive Medicine, Vol. 3, No. 4 31 December 2015 p167-173.

### Summary of Invention

Incidentally, in general, such an ablation system is required to improve convenience in use, for example. Accordingly, it is desirable to provide an ablation system that is able to improve convenience.

An ablation system according to one embodiment of the invention is described in claim 1.

In the ablation system according to one embodiment of the invention, the controller counts the number of times of breaks, and automatically ends the ablation by automatically stopping the supplying of the electric power from the power supply in a case where the number of times of breaks has reached the second threshold. Thus, it is possible to perform the effective ablation easily as compared with, for example, a case where the ablation is manually ended after, for example, visually confirming the number of times of breaks, a case where the ablation is automatically ended after a predetermined standby time has elapsed without confirming the number of times of breaks, or the like.

In the ablation system according to one embodiment of the invention, the power supply device may further include a display that displays a count value of the number of times of breaks. In this case, for example, an operator of the power supply device is able to know the count value of the number of times of breaks on an as-necessary basis, making it possible to further improve the convenience.

In the ablation system according to one embodiment of the invention, the controller may automatically resume the supplying of the electric power in the case where the number of times of breaks has not reached the second threshold (a first operation mode). In this case (in a case where the first operation mode is configured to be executed), the supplying of the electric power is automatically resumed, allowing a series of processes from the start to the end of the ablation to be performed automatically, and thereby making it possible to further improve the convenience.

Alternatively, the controller may resume the supplying of the electric power on the basis of an operation signal that is inputted in response to an operation performed by an operator in a case where the number of times of breaks has not reached the second threshold (a second operation mode). In this case (in a case where the second operation mode is configured to be executed), the resume of the supplying of the electric power itself is performed manually, allowing, for example, a timing of resuming the supplying of the electric power to be adjusted on an as-necessary basis, and thereby making it possible to further improve the convenience in this case as well.

In addition, the power supply device may be configured to be switchable between the first operation mode and the second operation mode. In this case, it is possible to switch the two types of operation modes on an as-necessary basis depending on, for example, application, a situation, etc., upon resuming the supplying the electric power, making it possible to further improve the convenience.

In the ablation system according to one embodiment of the invention, a liquid feeding device may be further provided that feeds a cooling liquid to the electrode needle, and the controller may also automatically stop feeding of the liquid after automatically ending the ablation, in the case where the number of times of breaks has reached the second threshold. In this case, it is possible to stop the feeding of the liquid at an appropriate timing as compared with a case where the feeding of the liquid is stopped manually after the end of the ablation, for example. As a result, it is possible to avoid a possibility that the affected site (the tissue) after the ablation is cooled by the liquid more than necessary, and to confirm a level of the ablation of the affected site more accurately, thereby making it possible to further improve the convenience.

In the ablation system according to one embodiment of the invention, the controller counts the number of times of breaks, and automatically ends the ablation by automatically stopping the supplying of the electric power from the power supply in a case where the number of times of breaks has reached the second threshold, making it possible to perform the effective ablation easily. Hence, it is possible to improve convenience upon using the ablation system.

### Brief Description of Drawings

[FIG. 1] FIG. 1 is a block diagram schematically illustrating an example of an overall configuration of an ablation system according to one embodiment of the invention.
[FIG. 2] FIG. 2 is a schematic diagram illustrating an example of levels of an ablation in an affected site resulting from the ablation.
[FIG. 3] FIG. 3 is a timing chart schematically illustrating an example of a break state and the number of times of breaks upon the ablation.
[FIG. 4] FIG. 4 is a flowchart illustrating an example of processes of the ablation according to the embodiment.
[FIG. 5] FIG. 5 is a schematic diagram illustrating an example of displaying performed on a display upon the ablation illustrated in FIG. 4.
[FIG. 6] FIG. 6 is a diagram illustrating an example of operation modes upon resuming of supplying of electric power illustrated in FIG. 4.

### Description of Embodiments

In the following, embodiments of the invention will be described in detail with reference to the drawings. The description will be made in the following order.
1. Embodiment (an example in which a controller provided in a power supply device performs automatic counting of the number of times of breaks, etc.)
2. Modification Examples

### <1. Embodiment>

### [Configuration]

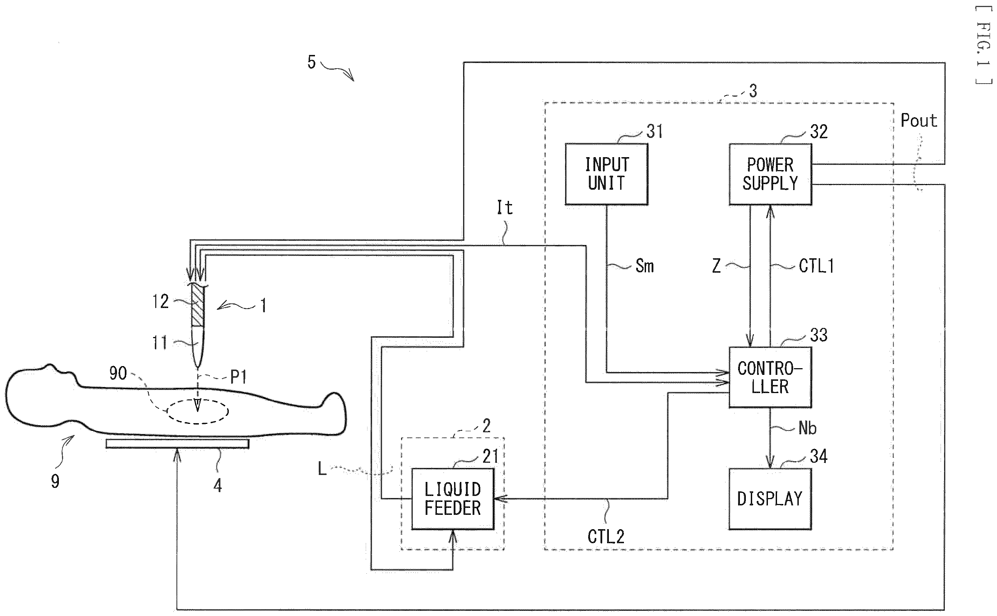

FIG. 1 is a block diagram schematically illustrating an example of an overall configuration of an ablation system (an ablation system 5) according to an embodiment of the invention. As illustrated by way of example in FIG. 1, the ablation system 5 is a system used upon treating an affected site 90 in a body of a patient 9, and performs a predetermined ablation on the affected site 90. The affected site 90 includes, for example, an affected site having a tumor such as a cancer (liver cancer, lung cancer, breast cancer, kidney cancer, thyroid cancer, etc.).

Referring to FIG. 1, the ablation system 5 includes an electrode needle 1, a liquid feeding device 2, and a power supply device 3. In addition, upon the ablation that uses the ablation system 5, a counter electrode plate 4 illustrated by way of example in FIG. 1 is also used on an as-needed basis.

### (A. Electrode Needle 1)

The electrode needle 1 is a needle that is to be percutaneously punctured into the affected site 90 in the body of the patient 9 as denoted by an arrow P1 in FIG. 1, for example. The electrode needle 1 is used upon the ablation, and includes an electrode 11 and a covering 12 as illustrated by way of example in FIG. 1. It is to be noted that a liquid L fed from a later-described liquid feeding device 2 circulates and flows in the inside of the electrode needle 1 (see FIG. 1).

The electrode 11 is a region of a needle-shaped structure configuring the electrode needle 1 in which an insulating coating is not provided, and functions as an electrode upon the ablation. The covering 12 is a region of the needle-shaped structure in which the insulating coating is provided. As illustrated in FIG. 1, the electrode 11 is disposed in the vicinity of a distal end of the electrode needle 1, and the covering 12 is disposed on the base end side of the electrode 11.

### (Liquid Feeding Device 2)

The liquid feeding device 2 feeds the cooling liquid L to the electrode needle 1. The liquid feeding device 2 includes a liquid feeder 21 as illustrated by way of example in FIG. 1. Examples of the cooling liquid L may include sterile water and sterile physiological saline.

The liquid feeder 21 feeds the liquid L to the electrode needle 1 on an as necessary basis, in accordance with a control by a later-described control signal CTL2. Specifically, the liquid feeder 21 so performs a feeding operation of the liquid L as to cause the liquid L to circulate (in a predetermined flow path) between the inside of the liquid feeding device 2 and the inside of the electrode needle 1, as illustrated by way of example in FIG. 1. In addition, as will be described later in detail, the feeding operation of the liquid L is executed or stopped in accordance with the control by the control signal CTL2. For example, the liquid feeder 21 includes a liquid pump or the like.

### (Power Supply Device 3)

The power supply device 3 supplies, between the electrode needle 1 and the counter electrode plate 4, electric power Pout (for example, high-frequency (RF: Radio Frequency) electric power) directed to performing of the ablation. The power supply device 3 also controls the feeding operation of the liquid L in the liquid feeding device 2. The power supply device 3 includes an input unit 31, a power supply 32, a controller 33, and a display 34, as illustrated in FIG. 1.

The input unit 31 is for inputting various setting values and an instruction signal (an operation signal Sm). The operation signal Sm is for instructing a later-described predetermined operation. The operation signal Sm is inputted from the input unit 31 in response to an operation performed by an operator (such as a technician) of the power supply device 3. However, for example, these various setting values may be set in the power supply device 3 in advance upon shipment of a product or the like, instead of being inputted in response to the operation performed by the operator. The setting value inputted by the input unit 31 is supplied to the later-described controller 33. The input unit 31 includes, for example, a predetermined dial, a button, a touch panel, or the like.

The power supply 32 supplies the electric power Pout between the electrode needle 1 and the counter electrode plate 4 in accordance with a later-described control signal CTL1. The power supply 32 includes a predetermined power supply circuit (such as a switching regulator). In a case where the electric power Pout includes high-frequency electric power, a frequency thereof is, for example, about 450 kHz to about 550 kHz (for example, 500 kHz).

The controller 33 controls the power supply device 3 as a whole and performs a predetermined arithmetic process. For example, the controller 33 includes a microcomputer or the like. Specifically, first, the controller 33 has a function of controlling, with the control signal CTL1, a supplying operation of the electric power Pout in the power supply 32 (an electric power supplying controlling function). The controller 33 also has a function of controlling, with the control signal CTL2, the feeding operation of the liquid L in the liquid feeding device 2 (the liquid feeder 21) (a liquid feeding controlling function).

The controller 33 is also supplied with temperature information It on an as-necessary basis. The temperature information It is measured by the electrode needle 1 (a temperature sensor such as a thermocouple disposed inside the electrode 11), as illustrated by way of example in FIG. 1. The controller 33 is supplied with a measured value of an impedance value Z (described later) from the power supply 32, as illustrated by way of example in FIG. 1.

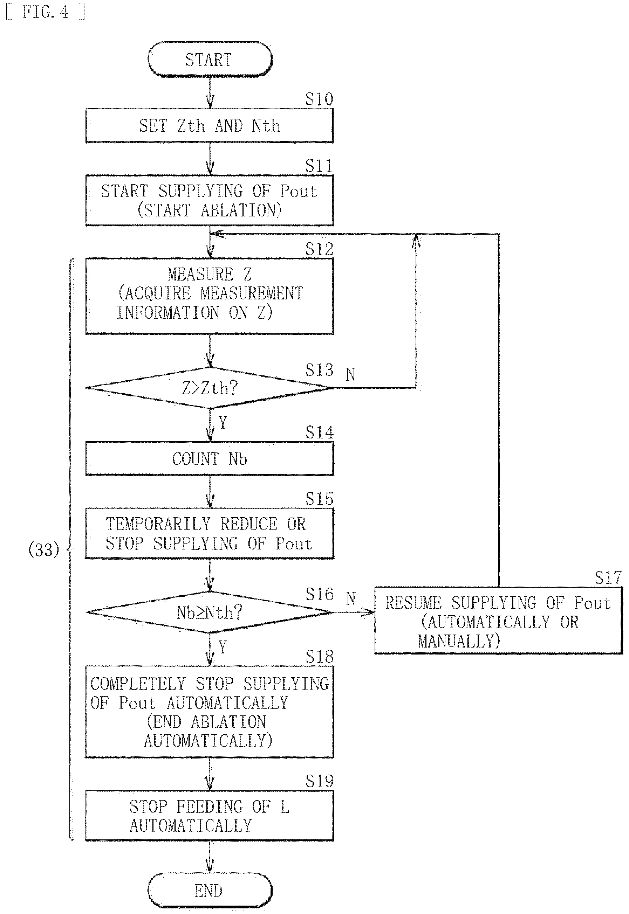

Note that details of control operations, etc., in the controller 33, including the electric power supplying controlling function and the liquid feeding controlling function, will be described later (FIGs. 4 to 6).

The display 34 is a section (a monitor) that displays various types of information and outputs the various types of information to the outside. Examples of information to be displayed may include: the above-described various setting values inputted from the input unit 31; various parameters supplied from the controller 33 (such as a count value of the number of times of breaks Nb to be described later); and the temperature information It supplied from the electrode needle 1. However, the information to be displayed is not limited to these pieces of information, and other information may be displayed instead of or in addition to other information. The display 34 includes a display of any of various types (such as a liquid crystal display, a CRT (Cathode Ray Tube) display, or an organic EL (Electro Luminescence) display).

### (Counter Electrode Plate 4)

The counter electrode plate 4 is used in a state of being mounted on a body surface of the patient 9 upon the ablation, as illustrated by way of example in FIG. 1. As will be described later in detail, high-frequency electricity is supplied (the electric power Pout is supplied) between the electrode needle 1 (the electrode 11) and the counter electrode plate 4 upon the ablation. Further, as will be described later, the impedance value Z between the electrode needle 1 (the electrode 11) and the counter electrode plate 4 is measured on an as-necessary basis upon the ablation, as illustrated in FIG. 1. The measured impedance value Z is supplied from the power supply 32 to the controller 33 in the power supply device 3.

### [Operation, Workings, and Effects]

### (A. Basic Operation)

In the ablation system 5, when, for example, the affected site 90 having a tumor such as a cancer is to be treated, a predetermined ablation is performed on the affected site 90 (see FIG. 1). In such an ablation, first, the electrode needle 1 is percutaneously punctured into the affected site 90 in the body of the patient 9 from the distal end side (the electrode 11 side), as indicated by the arrow P1 in FIG. 1, for example. Further, the electric power Pout (for example, the high-frequency electric power) is supplied between the electrode needle 1 and the counter electrode plate 4 from the power supply device 3 (the power supply 32), thereby performing the ablation of the affected site 90 based on Joule-heating.

Further, upon the ablation, the liquid L is so fed from the liquid feeding device 2 (the liquid feeder 21) to the electrode needle 1 as to cause the cooling liquid L to circulate (in a predetermined flow path) between the inside of the liquid feeding device 2 and the inside of the electrode needle 1 (see FIG. 1). As a result, a cooling operation (cooling) is performed on the electrode needle 1 upon the ablation. After the ablation is completed, the cooling operation is also stopped, following which a level of the ablation of the affected site is confirmed on the basis of the temperature information It measured by the electrode needle 1. For example, whether a tissue temperature of the affected site 90 is sufficiently increased is confirmed.

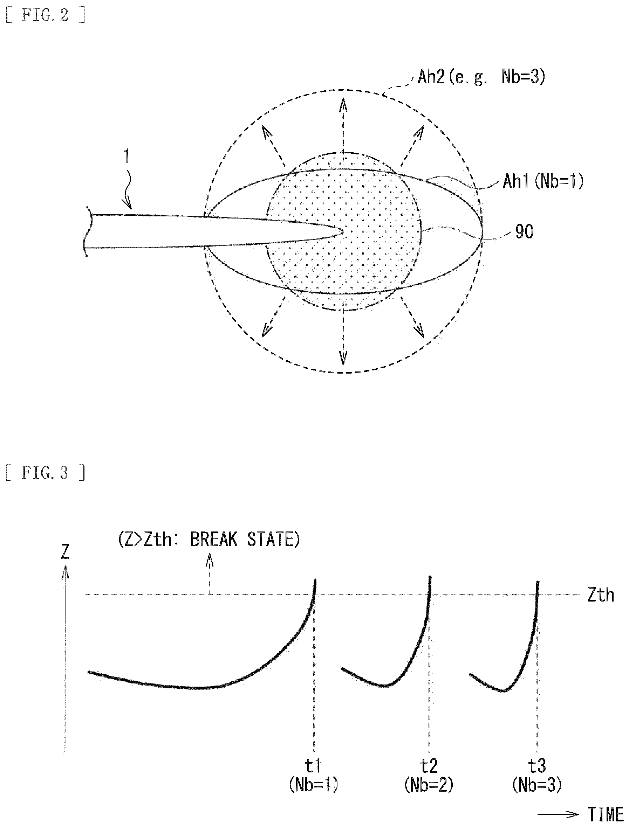

FIG. 2 schematically illustrates an example of levels of the ablation in the affected site 90 resulting from the ablation. Referring to FIG. 2, when the ablation is performed using the electrode needle 1 punctured in the affected site 90, a thermal coagulation region Ah1, which is initially in a rugby ball shape (an elliptical shape), gradually expands to be a substantially-spherical thermal coagulation region Ah2 (see a broken-line arrow in FIG. 2), for example. Thus, the isotropic ablation is performed on the affected site 90 as a whole, resulting in effective treatment of the affected site 90.

### (B. Break State and Number of Times of Breaks)

A description is given now of details of the ablation with reference to FIG. 3 in addition to FIGs. 1 and 2. FIG. 3 is a timing chart schematically illustrating an example of a break state and the number of times of breaks upon the ablation. Specifically, FIG. 3 illustrates, along a time axis, an example of a measured waveform of the impedance value Z between the electrode needle 1 (the electrode 11) and the counter electrode plate 4. It is to be noted that a threshold Zth of the impedance value Z illustrated in FIG. 3 corresponds to a specific example of a "first threshold" in the invention.

In general, as can be appreciated from an example illustrated in FIG. 3, the impedance value Z rapidly rises due to evaporation of a moisture in the tissue in the affected site 90 upon the ablation that uses the electrode needle. Such a rapid increase in the impedance value Z (an impedance rise) serves as an index of thermal coagulation of the tissue in the affected site 90, and thus serves as an indication of a stop timing upon the ablation. Specifically, a state in which the impedance value Z exceeds the predetermined threshold Zth (Z>Zth) is referred to as a "break state" (see FIG. 3). Further, the ablation (the supplying of the electric power Pout) is temporarily stopped when the break state is established, following which the ablation is resumed. Note that a moisture is supplied from the surrounding tissue into the tissue in the affected site 90 when the ablation is temporarily stopped, causing the impedance value Z to be decreased again consequently (see FIG. 3). Such an intermittent ablation is repeated a plurality of times to treat the affected site 90. For example, the above-described temporary stop time of the ablation (a standby time until the resume of the ablation) may be a predetermined time set in advance (for example, about 10 seconds to about 15 seconds), or may be a time until which the impedance value Z substantially returns to a value that is before the impedance value Z rises.

Specifically, in an example illustrated in FIG. 3, the ablation is temporarily stopped due to the establishment of the break state three times at timings t1, t2, and t3 with an elapse of time. The number of times of the break states in a case where the break state is repeated a plurality of times is hereinafter referred to as "the number of times of breaks Nb". That is, in an example of FIG. 3, the first break state is established at the timing t1 (Nb=1), the second break state is established at the timing t2 (Nb=2), and the third break state is established at the timing t3 (Nb=3).

Incidentally, in general, the number of times of breaks Nb of about 2 to 3 times (Nb=2 or Nb=3) is considered to be an indication in the treatment of the affected site 90 by the ablation that uses the electrode needle 1. That is, the thermal coagulation regions Ah1 and Ah2 illustrated in FIG. 2 as described above respectively correspond to Nb=1 and Nb=3 as an example.

### (C. Comparative Example)

In existing general techniques, the ablation is ended as follows upon the ablation based on the ablation system described above. Specifically, first, there is a technique (Comparative Example 1) in which the ablation is manually ended after, for example, an operator of the power supply device 3 has visually confirmed the number of times of breaks Nb (for example, about 2 to 3 times as described above). In addition, there is a technique (Comparative Example 2) in which the ablation is automatically ended by automatically stopping the supplying of the electric power Pout after a predetermined standby time (a fixed value) has elapsed, without confirming the number of times of breaks Nb.

However, in the technique according to the Comparative Example 1, the confirmation is made visually by the operator, for example. Accordingly, the count of the number of times of breaks Nb can become inaccurate due to, for example, overlooking of the break state. There is also a risk that extra time is consumed to perform the ablation. Because the transition to the break state at and after the second time is generally made in a short time (see FIG. 3), it can be said that such a risk is very high. In the technique according to the Comparative Example 2, a predetermined standby time is always required. Accordingly, the number of times of breaks Nb can increase more than necessary (for example, four or more times) within the ablation time. For these reasons, a greater number of break states are given to the affected site 90 than necessary upon the treatment by the ablation in the Comparative Examples 1 and 2 and the like, which consequently can increase the pain to be felt by the patient 9 and can increase the burden to be imposed on the patient 9 as well.

Here, the "pain" refers to the pain to be felt by the patient 9 upon the treatment. For example, it is said that the right shoulder, etc., often become painful as the related pain through the spinal nerve. It is to be noted that the impedance value Z suddenly rises in the break state, causing an output voltage to suddenly rise accordingly in a case where the electric power Pout is outputted at a constant power, for example. Further, a temperature at the affected site 90 tends to rise as well before the transition to the break state. Accordingly, the pain is said to be caused both by electrical and thermal occurrence factors.

In addition, depending on a type of the tumor in the affected site 90, there are the following example disadvantages if such pain increases. Specifically, for example, liver cancer generally requires repeated treatment due to its high rate of recurrence as compared with a cancer in any other organ. Under such circumstances, the memory of the pain felt by the patient 9 can prevent the patient 9 from receiving the next treatment. On the other hand, intensifying anesthesia upon the treatment is able to alleviate such pain; however, intensifying the anesthesia can hinder prediction of complications. For these reasons, it is ideal to minimize the pain while using the minimum amount of anesthesia. Accordingly, for the number of times of breaks Nb upon the treatment by the ablation, increasing the number of times of breaks Nb more than necessary is also undesirable as in the Comparative Examples 1 and 2 and the like.

Hence, it is difficult to perform the effective ablation and convenience upon using the ablation system can be impaired consequently in the Comparative Examples 1 and 2 and the like.

### (D. Ablation in Present Embodiment)

Therefore, the ablation system 5 according to the present embodiment performs the ablation on the basis of a technique described in detail below to thereby solve the problems in the Comparative Examples 1 and 2 and the like, as illustrated by way of example in FIG. 4. FIG. 4 is a flowchart illustrating an example of processes of the ablation in the ablation system 5 according to the present embodiment.

The ablation of the present embodiment first sets the above-described threshold Zth (a threshold of the impedance value Z) and a later-described threshold Nth (a threshold of the number of times of breaks Nb) (step S10 in FIG. 4). Specifically, setting values of the threshold Zth and the threshold Nth are each inputted from the input unit 31 in response to an operation performed by the operator of the power supply device 3, and are each supplied to the controller 33. The threshold Nth corresponds to a specific example of a "second threshold" in the invention.

For example, the threshold Nth may be three times (Nth=3) as described above with respect to the number of times of breaks Nb. Methods of defining the threshold Zth are roughly classified into a method of defining the threshold Zth by an absolute value (for example, Zth=about 120 [Ω]) and a method of defining the threshold Zth by a relative value (a method of defining the threshold Zth on the basis of how many Ω or how many % has increased). Further, as the method of defining the threshold Zth by the relative value, there are a method of defining the threshold Zth on the basis of the impedance value Z at the start of the ablation as a reference, and a method of defining the threshold Zth on the basis of the minimum value of the impedance value Z after the start of the ablation as a reference. Note that, as described previously, the setting values of the threshold Zth and the threshold Nth may be set in the power supply device 3 in advance, for example, upon shipment of a product or the like, instead of being inputted in response to an operation performed by the operator.

Next, the electric power Pout is supplied between the electrode needle 1 and the counter electrode plate 4 from the power supply device 3 (the power supply 32) to start the ablation of the affected site 90 (step S11). Specifically, to start the ablation, the operation signal Sm is inputted from the input unit 31 in response to an operation performed by the operator of the power supply device 3 and the operation signal Sm is supplied to the controller 33. That is, in this example, the ablation is started manually.

Next, when the ablation is started, the power supply 32 first measures the impedance value Z between the electrode needle 1 and the counter electrode plate 4 (step S12). In other words, the controller 33 acquires measurement information on the impedance value Z. When the thus-measured impedance value Z is supplied to the controller 33 from the power supply 32, the controller 33 then performs the following determination. That is, the controller 33 determines whether or not the impedance value Z is greater than the threshold Zth set in step S10 (whether or not Z>Zth is satisfied) (step S13). If it is determined that the impedance value Z is equal to or less than the threshold Zth (Z>Zth is not satisfied) (step S13: N), the process returns to the step S12, and the measurement of the impedance value Z is performed again.

If it is determined that the impedance value Z is greater than the threshold Zth (Z>Zth is satisfied) (step S13: Y), it means that the break state described above is established. In this case, the controller 33 then automatically counts the number of times of the break states (the number of times of breaks Nb) (step S14). The count value of the number of times of breaks Nb is stored on an as-necessary basis in, for example, various storage media provided in the controller 33.

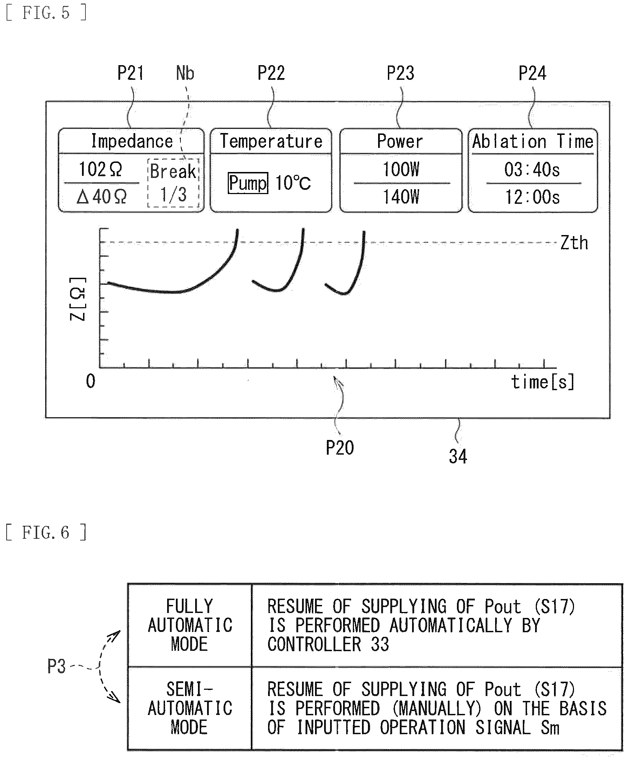

The count value of the number of times of breaks Nb and the like are displayed on the display 34 of the power supply device 3 on an as-necessary basis, as schematically illustrated by way of example in FIG. 5. Specifically, first, as schematically illustrated in FIG. 3 as well, the measured waveform of the impedance value Z is displayed on the display 34 along the time axis (see symbol P20 in FIG. 5). In addition, in an example of the display 34, a current value of the impedance value Z (Impedance: see symbol P21), the temperature information It (Temperature: see symbol P22), an output value of the electric power Pout (Power: see symbol P23), and information on the ablation time (Ablation Time: see symbol P24) are each further displayed. Moreover, in an example of the display 34, the current value of the impedance value Z (see symbol P21) and the count value of the number of times of breaks Nb are displayed together as illustrated in FIG. 5.

Next, the controller 33 temporarily reduces or stops the supplying of the electric power Pout from the power supply 32 by using the control signal CTL1 to thereby temporarily stop the ablation (step S15). As a result, the impedance value Z decreases again as described above, allowing for exiting from the break state.

Next, the controller 33 determines whether or not the number of times of breaks Nb counted in step S14 is equal to or greater than the threshold Nth set in step S10 (whether or not Nb≥Nth is satisfied) (step S16). If it is determined that the number of times of breaks Nb is less than the threshold Nth (Nb≥Nth is not satisfied) (step S16: N), the supplying of the electric power Pout (the ablation) is automatically or manually resumed thereafter (step S17).

FIG. 6 illustrates an example of operation modes upon resuming the supplying of the electric power Pout (step S17). Referring to FIG. 6, as the operation modes in such an occasion, there are two types of operation modes including a "fully automatic mode" and a "semi-automatic mode", for example. The fully automatic mode corresponds to a specific example of a "first operation mode" in the invention, and the semi-automatic mode corresponds to a specific example of a "second operation mode" in the invention.

In the fully automatic mode, if the number of times of breaks Nb has not reached the threshold Nth (step S16: N), the controller 33 automatically resumes the supplying of the electric power Pout (the ablation) (step S17). Specifically, the controller 33 automatically resumes the supplying of the electric power Pout from the power supply 32 by using the control signal CTL1 described above. That is, the supplying of the electric power Pout is resumed automatically in the fully automatic mode.

In the semi-automatic mode, if the number of times of breaks Nb has not reached the threshold Nth (step S16: N), the controller 33 resumes the supplying of the electric power Pout (the ablation) on the basis of the operation signal Sm inputted in response to an operation performed by the operator of the power supply device 3 (step S17). In other words, the supplying of the electric power Pout is resumed manually in the semi-automatic mode.

Further, in the present embodiment, the power supply device 3 may be configured to be switchable between the two types of operation modes (the "fully automatic mode" and the "semi-automatic mode"), for example (see a broken-line arrow P3 illustrated in FIG. 6). That is, for example, these two types of operation modes may be switched on an as-necessary basis on the basis of the operation signal Sm inputted in response to an operation performed by the operator of the power supply device 3.

After the supplying of the electric power Pout (the ablation) is resumed, the process returns to step S12 to measure the impedance value Z again. It is to be noted that the impedance value Z is measurable continuously even in the break state, if the supplying of the electric power Pout is temporarily "reduced" in step S15 described above. If the supplying of the electric power Pout is temporarily "stopped" in step S15, the measurement of the impedance value Z is not performed in the break state.

If it is determined in step S16 that the number of times of breaks Nb is equal to or greater than the threshold Nth (Nb≥Nth is satisfied) (step S16: Y), the controller 33 performs the following control thereafter. That is, if the number of times of breaks Nb has reached the threshold Nth (step S16: Y), the controller 33 automatically ends the ablation by automatically stopping (completely stopping) the supplying of the electric power Pout from the power supply 32 (step S18). Specifically, the controller 33 automatically stops the supplying of the electric power Pout by using the control signal CTL1 described above. As a result, the ablation of the affected site 90 is automatically ended by the controller 33.

Next, after the controller 33 has automatically ended the ablation in this manner (step S18), the controller 33 automatically stops the feeding of the cooling liquid L fed from the liquid feeding device 2 as well (step S19). Specifically, the controller 33 automatically stops the feeding of the liquid L fed from the liquid feeder 21 by using the control signal CTL2 described above. Thus, the circulation of the liquid L between the inside of the liquid feeding device 2 and the inside of the electrode needle 1 is stopped (see FIG. 1), and the cooling operation (the cooling) performed on the electrode needle 1 is stopped. This completes the series of processes (an example of processes of the ablation according to the present embodiment) illustrated in FIG. 4.

### (E. Workings and Effects)

As described above, in the ablation system 5 according to the present embodiment, the controller 33 performs the following control upon the ablation. That is, first, the controller 33 measures the impedance value Z between the electrode needle 1 and the counter electrode plate 4 (step S12 in FIG. 4), and counts the number of times of the break states (the number of times of breaks Nb) in which the impedance value Z has exceeded the threshold Zth (step S14). In a case where the number of times of breaks Nb has reached the threshold Nth, the controller 33 automatically ends the ablation by automatically stopping the supplying of the electric power Pout (step S18).

Thus, the present embodiment achieves the following. That is, as compared with, for example, a case where the ablation is manually ended after, for example, visually confirming the number of times of breaks Nb as described above (Comparative Example 1), a case where the ablation is automatically ended after a predetermined standby time has elapsed without confirming the number of times of breaks Nb as described above (Comparative Example 2), or the like, it is possible to perform the effective ablation easily. Hence, the present embodiment makes it possible to improve convenience upon using the ablation system 5 as compared with the Comparative Examples 1 and 2 and the like.

In addition, in the present embodiment, the power supply device 3 is provided with the display 34 that displays the count value of the number of times of breaks Nb (see FIG. 5). Thus, for example, the operator of the power supply device 3 is able to know the count value of the number of times of breaks Nb on an as-necessary basis. Hence, it is possible to further improve the convenience.

Further, in the present embodiment, the following is achieved in a case where the controller 33 automatically resumes the supplying of the electric power Pout (the ablation) upon resuming the supplying of the electric power Pout (the ablation) (step S17 in FIG. 4), i.e., in a case where the "fully automatic mode" illustrated in FIG. 6 is executed. That is, in the "fully automatic mode", the supplying of the electric power Pout is automatically resumed, allowing the series of processes from the start to the end of the ablation (the processes of steps S12 to S19 in FIG. 4) to be performed automatically. Hence, it is possible to further improve the convenience.

Further, in the present embodiment, the following is achieved in a case where the supplying of the electric power Pout is resumed on the basis of the operation signal Sm inputted in response to an operation performed the operator of the power supply device 3 upon resuming the supplying of the electric power Pout (in a case where the "semi-automatic mode" illustrated in FIG. 6 is executed). That is, the resume of the supplying of the electric power Pout itself is performed manually, allowing, for example, a timing of resuming the supplying of the electric power Pout to be adjusted on an as-necessary basis. Hence, it is possible to further improve the convenience in this case as well.

In addition, in the present embodiment, the power supply device 3 is configured to be switchable between the two types of operation modes (the "fully automatic mode" and the "semi-automatic mode") (see the arrow P3 in FIG. 6), making is possible to achieve the following. That is, it is possible to switch the two types of modes on an as-necessary basis depending on, for example, application, a situation, etc., upon resuming the supplying the electric power Pout (the ablation) (step S17 in FIG. 4). Hence, it is possible to further improve the convenience.

Moreover, in the present embodiment, the controller 33 also automatically stops the feeding of the liquid L (step S19) after automatically ending the ablation (step S18 in FIG. 4) in a case where the number of times of breaks Nb has reached the threshold Nth, making is possible to achieve the following. That is, it is possible to stop the feeding of the liquid L at an appropriate timing as compared with, for example, a case where the feeding of the liquid L is stopped manually after the end of the ablation (Comparative Example 3). As a result, it is possible to avoid a possibility that the affected site 90 (the tissue) after the ablation is cooled by the liquid L more than necessary, and to confirm a level of the ablation of the affected site 90 more accurately. Hence, the present embodiment makes it is possible to further improve the convenience as compared with the Comparative Example 3 described above.

### <2. Modification Examples>

Although the invention has been described with reference an embodiment, the invention is not limited to the embodiment but may be modified in a wide variety of ways.

For example, materials, etc., of the respective members described in the foregoing embodiment are non-limiting, and other materials may be employed. In addition, although the electrode needle 1 has been described with specific reference to the configuration thereof in the foregoing embodiment, it is not necessary to include all of the components. Alternatively, any other component may be further included as well. In addition, values, ranges, magnitude relationships, etc., of the various parameters described in the foregoing embodiment are not limited to those described in the foregoing embodiment, and other values, ranges, magnitude relationships, etc., may be employed.

In addition, the forgoing embodiment has been described with specific reference to the block configuration of the liquid feeding device 2 and the power supply device 3. However, it does not necessarily have to include each of the blocks described in the foregoing embodiment, or any other block may be further included as well. Further, the ablation system 5 as a whole may further include any other device in addition to the devices described in the foregoing embodiment.

Further, the control operation (an ablation technique) in the controller 33, including the electric power supplying controlling function and the liquid feeding controlling function, has been described specifically in the foregoing embodiment. However, control techniques (the ablation technique) related to the electric power supplying controlling function, the liquid feeding controlling function, etc., are not limited to the techniques described in the foregoing embodiment. Specifically, for example, although the threshold Nth (the threshold of the number of times of breaks Nb) has been described by referring to an example in which the threshold Nth is mainly three times (Nth=3) in the foregoing embodiment, it is not limited thereto. The threshold Nth may be any number of times, such as one time (Nth=1), two times (Nth=2), or four or more times (Nth≥4).

The series of processes described in the foregoing embodiment may be performed by hardware (circuit) or software (program). In a case where the processes are to be performed by the software, the software includes a program group that causes a computer to execute each function. When using each program, each program may be incorporated in the computer in advance, or may be installed in the computer from a network or a recording medium.

Furthermore, the various examples described above may be applied in any combination.

## Claims

1. An ablation system (5) comprising:
an electrode needle (1) to be percutaneously punctured into an affected site (90) in a body; and
a power supply device (3) including a power supply (32) and a controller (33), the power supply (32) supplying electric power (Pout) between the electrode needle (1) and a counter electrode plate (4), the electric power (Pout) being directed to performing of an ablation, the controller (33) controlling a supplying operation of the electric power (Pout) in the power supply (32),
wherein
the controller (33): measures an impedance value (Z) between the electrode needle (1) and the counter electrode plate (4) upon the ablation; counts the number of times of breaks (Nb) upon the ablation and temporarily reduces or stops the supplying of the electric power, in a case where a break state is established, the break state being established when the impedance value has exceeded a predetermined first threshold that is set in advance, the number of times of breaks (Nb) being the number of times in which a transition is made to the break state,
automatically resumes the supplying of the electric power, in a case where the number of times of breaks has not reached a second threshold, and
automatically ends the ablation by automatically stopping supplying of the electric power (Pout), in a case where the number of times of breaks (Nb) has reached the second threshold (Nth).

2. The ablation system (5) according to claim 1, wherein the power supply device (3) further includes a display (34) that displays a count value of the number of times of breaks (Nb).

3. The ablation system (5) according to any of claims 1 or 2, further comprising a liquid feeding device (2) that feeds a cooling liquid (L) to the electrode needle (1), wherein
the controller (33) also automatically stops feeding of the liquid (L) after automatically ending the ablation, in the case where the number of times of breaks (Nb) has reached the second threshold (Nth).

## Patentansprüche

1. Ablationssystem (5), umfassend:
eine Elektrodennadel (1), die perkutan in eine betroffene Stelle (90) in einem Körper punktiert wird; und
eine Stromversorgungsvorrichtung (3) mit einer Stromversorgung (32) und einer Steuerung (33), wobei die Stromversorgung (32) elektrischen Strom (Pout) zwischen der Elektrodennadel (1) und einer Gegenelektrodenplatte (4) bereitstellt, wobei der elektrische Strom (Pout) auf das Durchführen einer Ablation gerichtet ist, wobei die Steuerung (33) einen Bereitstellungsvorgang des elektrischen Stroms (Pout) in der Stromversorgung (32) steuert,
wobei
die Steuerung (33): einen Impedanzwert (Z) zwischen der Elektrodennadel (1) und der Gegenelektrodenplatte (4) bei der Ablation misst; die Anzahl der Unterbrechungen (Nb) bei der Ablation zählt und vorübergehend die Bereitstellung des elektrischen Stroms reduziert oder stoppt, falls ein Unterbrechungszustand hergestellt wird, wobei der Unterbrechungszustand hergestellt wird, wenn der Impedanzwert einen vorbestimmten ersten Schwellenwert überschritten hat, der im Voraus festgelegt wird, wobei die Anzahl der Unterbrechungen (Nb) die Anzahl der Male ist, in denen ein Übergang in den Unterbrechungszustand erfolgt,
automatisch die Bereitstellung des elektrischen Stroms wieder aufnimmt, falls die Anzahl der Unterbrechungen einen zweiten Schwellenwert nicht erreicht hat, und
automatisch die Ablation beendet, indem die Bereitstellung des elektrischen Stroms (Pout) automatisch gestoppt wird, falls die Anzahl der Unterbrechungen (Nb) den zweiten Schwellenwert (Nth) erreicht hat.

2. Ablationssystem (5) nach Anspruch 1, wobei die Stromversorgungsvorrichtung (3) ferner eine Anzeige (34) aufweist, die einen Zählwert der Anzahl der Unterbrechungen (Nb) anzeigt.

3. Ablationssystem (5) nach einem der Ansprüche 1 oder 2, ferner eine Flüssigkeitszufuhrvorrichtung (2) umfassend, die der Elektrodennadel (1) eine Kühlflüssigkeit (L) zuführt, wobei
die Steuerung (33) ebenfalls automatisch die Zufuhr der Flüssigkeit (L) stoppt, nachdem die Ablation automatisch beendet wurde, falls die Anzahl der Unterbrechungen (Nb) den zweiten Schwellenwert (Nth) erreicht hat.

## Revendications

1. Système d'ablation (5) comprenant :
une aiguille électrode (1) destinée à perforer par voie percutanée un site affecté (90) dans un corps ; et
un dispositif d'alimentation (3) comportant une alimentation (32) et un dispositif de commande (33), l'alimentation (32) fournissant de l'énergie électrique (Pout) entre l'aiguille électrode (1) et une plaque de contre-électrode (4), l'énergie électrique (Pout) étant destinée à effectuer une ablation, le dispositif de commande (33) commandant une opération d'alimentation de l'énergie électrique (Pout) dans l'alimentation (32),
dans lequel :
le dispositif de commande (33) : mesure une valeur d'impédance (Z) entre l'aiguille électrode (1) et la plaque de contre-électrode (4) lors de l'ablation ; compte le nombre de ruptures (Nb) lors de l'ablation et réduit ou arrête temporairement l'alimentation en énergie électrique, dans le cas où un état de rupture est établi, l'état de rupture étant établi lorsque la valeur d'impédance a dépassé un premier seuil prédéterminé qui est fixé à l'avance, le nombre de ruptures (Nb) étant le nombre de passages à l'état de rupture,
reprend automatiquement l'alimentation en énergie électrique, dans le cas où le nombre de ruptures n'a pas atteint un second seuil, et
arrête automatiquement l'ablation en arrêtant automatiquement l'alimentation en énergie électrique (Pout), dans le cas où le nombre de ruptures (Nb) a atteint le second seuil (Nth).

2. Système d'ablation (5) selon la revendication 1, dans lequel le dispositif d'alimentation en énergie (3) comporte en outre un afficheur (34) qui affiche une valeur de comptage du nombre de ruptures (Nb).

3. Système d'ablation (5) selon l'une quelconque des revendications 1 et 2, comprenant en outre un dispositif d'alimentation en liquide (2) qui introduit un liquide de refroidissement (1) dans l'aiguille électrode (1), dans lequel
le dispositif de commande (33) arrête également automatiquement l'introduction du liquide (L) après la fin automatique de l'ablation, dans le cas où le nombre de ruptures (Nb) a atteint le second seuil (Nth).
